# EUROPÄISCHE PATENTSCHRIFT

(11) **EP 1 716 091 B1**
(45) Veröffentlichungstag und Bekanntmachung des Hinweises auf die Patenterteilung: **18.02.2009**
(21) Anmeldenummer: 05715270.4
(22) Anmeldetag: 08.02.2005
(51) Int. Cl.: C07C 29/149, C07C 31/22, C07C 213/00, C07C 215/08, C07D 307/33

(54) **VERFAHREN ZUR HERSTELLUNG OPTISCH AKTIVER ALKOHOLE ODER CARBONSÄUREN**
METHOD FOR PRODUCING OPTICALLY ACTIVE ALCOHOLS OR CARBOXYLIC ACIDS
PROCEDE DE PRODUCTION DE D'ALCOOLS OU D'ACIDES CARBOXYLIQUES OPTIQUEMENT ACTIFS

(30) Priorität: 13.02.2004 DE 102004007499
(43) Veröffentlichungstag der Anmeldung: 02.11.2006
(73) Patentinhaber: BASF SE, 67056 Ludwigshafen (DE)
(72) Erfinder: URTEL, Heiko, 68535 Edingen-Neckarhausen (DE); RÖSCH, Markus, 55276 Dienheim (DE); HAUNERT, Andrea, 68163 Mannheim (DE); SCHUBERT, Markus, 67063 Ludwigshafen (DE)
(86) Internationale Anmeldenummer: PCT/EP2005/001235
(87) Internationale Veröffentlichungsnummer: WO 2005/077871

(56) Entgegenhaltungen:
- WO-A-99/38824
- WO-A-99/38838
- WO-A-20/04022522

## Beschreibung

Die vorliegende Erfindung betrifft ein Verfahren zur Herstellung optisch aktiver Hydroxy-, Alkoxy-, Amino-, Alkyl-, Aryl- oder Chlor-substituerter Alkohole oder Hydroxycarbonsäuren mit 3 bis 25 Kohlenstoffatomen oder deren Säurederivate oder Cyclisierungsprodukte durch Hydrierung der entsprechend substituierten optisch aktiven Mono- oder Dicarbonsäuren oder deren Säurederivate.

Die genannten Zietverbindungen stellen für die pharmazeutische und kosmetische Industrie wertvolle Intermediate zur Herstellung von Arzneimitteln, Duftstoffen und anderen organischen Feinchemikalien dar, die kostengünstig nur schwer zugänglich sind.

Die EP-A 0696575 beschreibt ein Verfahren zur Herstellung von optisch aktiven Aminoalkoholen durch Hydrierung der ensprechenden Aminosäuren in Gegenwart von mit , Wasserstoff reduzierten Ru-haltigen Katalysatoren bei Temperaturen von 50 bis 150°C und Drucken von 5 bis 300 bar.

Die EP-A 0717023 betrifft ein Verfahren zur Herstellung von optisch aktiven Alkoholen durch Reduktion der entsprechenden optisch aktiven Carbonsäuren in Gegenwart von mit Wasserstoff reduzierten Ru-haltigen Katalysatoren bei Temperaturen < 160°C und Drücken < 250 bar.

Die WO 99/38838 beschreibt ein Verfahren zur Herstellung von optisch aktiven Aminoalkoholen durch katalytische Hydrierung der entsprechenden Aminosäuren mit bi- bzw. trimetallischen ungeträgerten oder geträgerten Ru-Katalysatoren unter Zusatz von Säure.

In der WO 99/38613 wird die Herstellung von ungeträgerten Hydrierkatalysatoren, die Ruthenium und wenigstens ein weiteres Metall mit einer Ordnungszahl von 22 bis 83 enthalten. Mit diesen Katalysatoren lassen sich Carbonsäuren und deren Derivate bei mildern Bedingungen hydrieren. Bei enantiomerenreinen Substraten beträgt der erzielbare Enantiomerenüberschuss maximal 98.8% bei Ausbeuten unter 80 %.

Die WO 99/38824 beschreibt ein Verfahren zur Herstellung von optisch aktiven Alkoholen durch Reduktion von optisch aktiven Carbonsäuren in Gegenwart von mit Wasserstoff reduzierten Ru-haltigen Katalysatoren, die mindestens ein weiteres Metall mit den Ordnungszahlen im Bereich von 22 bis 83 enthalten.

In der EP-A 1051388 werden ungeträgerte Ru/Re-Suspensionskatalysatoren beschrieben, mit denen sich chirale α-Amino- bzw. α-Hydroxysäuren bei 60 bis 100°C und 200 bar Wasserstoffdruck zu den entsprechenden chiralen Alkoholen reduzieren lassen.

Aus der US-4,659-686 geht hervor, dass mit Alkali- oder Erdalkalidotierten Katalysatoren, die ein Pt-Gruppen-Metall und Re auf Kohle enthalten, bei der Hydrierung von Apfelsäure als Reaktionsprodukte Tetrahydrofuran (THF) und/oder Butandiol (BDO) entstehen, 1,2,4-Butantriol wird mit diesen Katalysatoren nicht gefunden.

Die EP-A 147 219 beschreibt Pd-Re-Katalysatoren und ihre Verwendung in einem Verfahren zur Herstellung von THF und BDO. Aus Beispiel 39 geht hervor, dass die Hydrierung von Äpfelsäure bei 200°C und 170 bar in Ausbeuten von 66 % THF und von 21 % zu BDO führt. 1,2,4.Butantriol wird nicht gefunden.

In Adv. Synth. Catal. 2001, 343, Nr. 8 wird die Verwendung des Nishimura-Katalysators (Rh-Pt-Oxid) für die racemisierungsfreie Hydrierung von α-Aminosäureestern und α-Hydroxycarbonsäureestern beschrieben. Dort werden allerdings große Mengen (10 Gew.%) des teuren Katalysatorsystems benötigt. Des weiteren müssen die freien Carbonsäuren in einem weiteren Syntheseschritt zunächst in die entsprechenden Ester überführt werden.

Die WO2004/022522 betrifft ein Verfahren zur Herstellung optisch aktiver 2-Amino-, 2-Chlor-, 2-Hydroxy- oder 2-Alkoxy-1-Alkanole durch katalytische Hydrierung entsprechender optisch aktiver 2-Amino-, 2-Chlor-, 2-Hydroxy- und 2-Alkoxycarbonsäuren oder ihrer Säurederivate, wobei man die Hydrierung in Gegenwart von Palladium und Rhenium oder Platin und Rhenium enthaltenden Katalysatoren durchführt.

Ein Problem bei der Verwendung Ru-haltiger Katalysatoren bei der Hydrierung von Carbonsäuren besteht darin, dass diese zur starken Decarbonylierung der eingesetzten Edukte bzw. erhaltenen Produkte unter Freisetzung von Kohlenmonoxid neigen. Neben dem damit verbundenen starken Druckanstieg stellt die Reduktion des freigesetzten Kohlenmonoxids zum Methan ein großes Sicherheitsrisiko dar.

Aufgabe der vorliegenden Erfindung war es, ein Verfahren zur Hydrierung optisch aktiver Carbonsäuren oder deren Säurederivate zu den entsprechenden optisch aktiven Alkoholen bereitzustellen, bei dem die unerwünschte Decarbonylierung der eingesetzten Edukte bzw. der gebildeten Produkte weitestgehend vermieden wird.

Die Aufgabe wurde erfindungsgemäß gelöst durch die Bereitstellung eines Verfahrens zur Herstellung optisch aktiver Hydroxy-, Alkoxy-, Amino-, Alkyl-, Aryl- oder Chlor-substituerter Alkohole oder Hydroxycarbonsäuren mit 3 bis 25 Kohlenstoffatomen oder deren Säurederivate oder Cyctisierungsprodukte durch Hydrierung der entsprechend substituierten optisch aktiven Mono- oder Dicarbonsäuren oder deren Säurederivate in Gegenwart eines Katalysators, dessen Aktivkomponente aus Rhenium besteht oder Rhenium und mindestens ein weiteres Element ausgewählt aus der Gruppe der Elemente Pd, Pt, Rh, Ir, Cu, Ag, Au, Cr, Mo, W, Co und Ni enthält, mit der Maßgab dass,
im Falle der Herstellung optisch aktiver 2-Amino-, 2-Chlor-, 2-Hydroxy- und 2-Alkoxy-1-Alkanole durch katalytische Hydrierung entsprechender optisch aktiver 2-Amino-, 2-Chlor-, 2-Hydroxy- und 2-Alkoxycarbonsäuren oder ihrer Säurederivate das mindestens eine weitere Element nicht Palladium oder Platin ist.

Das erfindungsgemäße Verfahren eignet sich zur Hydrierung von optisch aktiven Mono- oder Dicarbonsäuren mit 3 bis 25, bevorzugt mit 3 bis 12 Kohlenstoffatomen, die geradkettig, verzweigt oder cyclisch sein können und mindestens einen, üblicherweise 1 bis 4, an je ein asymmetrisch substituiertes Kohlenstoffatom gebundenen Substituenten aufweisen. Das Verfahren eignet sich gleichermaßen zur Hydrierung von Säurederivaten der genannten substituierten Carbonsäuren. Dabei ist unter dem Begriff Säurederivate hier wie im gesamten Rahmen der vorliegenden Erfindung zu verstehen, dass die Säurefunktion in Form eines Esters, eines Halbesters, eines Anhydrids oder eines Amids, bevorzugt in Form eines Esters oder Halbesters vorliegt.

Unter optisch aktiven Verbindungen sind im Rahmen der vorliegenden Erfindung solche Verbindungen zu verstehen, die in der Lage sind, als solche oder in gelöster Form die Polarisationsebene des durchtretenden linear polarisierten Lichtes zu drehen. Bei Verbindungen mit einem stereogenen Zentrum handelt es sich dabei um nichtracemische Gemische der beiden Enantiomere. d.h. um Gemische, in denen die beiden Enantiomere zu nicht gleichen Teilen vorliegen. Im Falle der Umsetzung von Verbindungen mit mehr als einem Stereozentrum können verschiedene Diasteromere erhalten werden, die jeweils für sich betrachtet als optisch aktive Verbindungen aufzufassen sind.

Als an asymmetrisch substituierte Kohlenstoffatome gebundene Substituenten kommen in Betracht: Hydroxy-, Alkoxy-, Amino-, Alkyl-, Aryl- oder Chlor-Substituenten, wobei unter Alkoxy-Subsituenten insbesondere solche zu verstehen sind, deren an das Sauerstoffatom gebundener organischer Rest 1 bis 8 Kohtensfoffatome aufweist, Amino-Substituenten in Form des freien Amins oder bevorzugt in protonierter Form aus Ammoniumsalz und gegebenenfalls mit einem oder zwei organischen Resten mit je 1 bis 5 Kohlenstoffatomen vorliegen können, die Alkyl- Substituenten 1 bis 10 Kohlenstoffatome und die Aryl-Substituenten 3 bis 14 Kohlenstoffatome aufweisen und ihrerseits unter den Reaktionsbedingungen stabile Substituenten tragen können und die Aryl-Substituenten auch 1 bis 3 Heteroatome wie beispielsweise N, S und /oder O aufweisen können.

Die genannten Substituenten können prinzipiell an jeder möglichen Stelle der umzusetzenden Mono- oder Dicarbonsäure angebunden sein. Bevorzugte Substrate sind im Rahmen der vorliegenden Erfindung solche, die mindestens einen der genannten Substituenten aufweisen, der an ein asymmetrisches Kohlenstoffatom in α- oder β-Position, besonders bevorzugt in α-position zu der zu hydrierenden Säurefunktion aufweisen.

Die erfindungsgemäße Hydrierreaktion kann im Falle der Umsetzung von Dicarbonsäuren wahlweise so geführt werden, dass entweder nur eine oder beide der im Substratmolekül vorhandenen Carbonsäurefunktionen bzw. Carbonsäurederivatfunktionen zu den Hydroxyfunktionen hydriert werden.

Beispielsweise eignet sich das erfindungsgemäße Verfahren zur Umsetzung optisch aktiver Carbonsäuren oder deren Säurederivate der Formel 1, 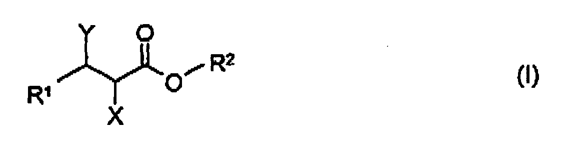 in der die Reste folgende Bedeutung haben:
- R¹:: Geradkettiges und verzweigtes C₁-C₁₂Alkyl, C₇-C₁₂-Aralkyl oder C₆-C₁₄-Aryl, wobei die genannten Reste durch NR³R⁴, OH, COOH und/oder weitere, unter den Reaktionsbedingungen stabile Gruppen substituiert sein können,
- R²:: Wasserstoff, geradkettiges oder verzweigtes C₁-C₁₂Alkyl oder C₃-C₈-Cycloalkyl,
- X, Y:: Unabhängig voneinander Wasserstoff, Chlor, NR⁵R⁶ oder OR⁷, geradkettiges oder verzweigtes C₁-C₁₀-Alkyl oder C₆-C₁₄-Aryl mit der Maßgabe dass mindestens einer der Reste X oder Y nicht Wasserstoff ist,
- X und R¹ oder Y und R¹:: Gemeinsam auch einen 5- bis 8-gliedrigen Cyclus darstellen können,
- R³, R⁴, R⁵ und R⁶:: Unabhängig voneinander jeweils Wasserstoff, geradkettiges und verzweigtes C₁-C₁₂ Alkyl, C₇-C₁₂-Aralkyl, C₆-C₁₄-Aryl, C₃-C₈-Cycloalkyl oder C₃-C₈-Cycloalkyl, in dem eine CH₂-Gruppe durch O oder NR⁸ ersetzt ist,
- R³ und R⁴ sowie R⁵ und R⁶:: Unabhängig voneinander jeweils gemeinsam auch -(CH₂)ₘ-, wobei m eine ganze Zahl von 4 bis 7 bedeutet,
- R¹ und R⁶:: Gemeinsam auch -(CH₂)ₙ-, wobei n einer ganzen Zahl von 2 bis 6 entspricht,
- R⁷:: Wasserstoff, geradkettiges oder verzweigtes C₁-C₁₂-Alkyl oder C₃-C₈-Cycloalkyl,
- R¹ und R⁷:: Gemeinsam auch -(CH₂)ₙ-, wobei n einer ganzen Zahl von 2 bis 6 entspricht und
- R⁸:: Wasserstoff, geradkettiges oder verzweigtes C₁-C₁₂-Alkyl, C₇-C₁₂-Aralkyl oder C₈-C₁₄-Aryl,
oder deren Säureanhydride zu den entsprechenden optisch aktiven Alkoholen,

Die Reste R¹ können breit variiert werden und auch mehrere, z. B. 1 bis 3 unter den Reaktionsbedingungen stabile Substituenten wie NR³R⁴, OH und/oder COOH tragen.

Beispielhaft seien folgende Reste für R¹ genannt
C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2.2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl,
C₁-C₁₂-Alkyl wie C₁-C₈-Alkyl (vorstehend genannt) oder unverzweigtes oder verzweigtes Heptyl, Octyl, Nonyl, Decyl, Undecyl oder Dodecadecyl,
C₁-C₁₂-Aralkyl wie Phenylmethyl, 1-Phenylethyl 2-Phenylethyl, 1-Phenylpropyl, 2-Phenylpropyl oder 3-Phenylpropyl,
C₆-C₁₄-Aryl wie Phenyl, Naphthyl oder Anthracenyl, wobei die aromatischen Reste Substituenten wie NR⁹R¹⁰, OH und/oder COOH tragen können.

Für R² seien beispielhaft folgende Bedeutungen genannt:
Wasserstoff, geradkettiges oder verzweigtes C₁-C₁₂-Alkyl (wie vorstehend genannt) oder C₃-C₈-Cycloalkyl wie z. B. Cyclopropyl, Cyclobutyl, Cyclopentyl, Cyclohexyl, Cycloheptyl und Cyclooctyl.

Anstelle der Carbonsäureester können auch die Säureanhydride als Carbonsäurederivate eingesetzt werden.

Die Reste X und Y stehen unabhängig voneinander für Chlor, NR⁵R⁶ oder OR⁷, wobei R⁵ und R⁶, genauso wie R³ und R⁴, bzw. R⁹ und R¹⁰ unabhängig voneinander jeweils für Wasserstoff, geradkettiges und verzweigtes C₁-C₁₂-Alkyl, insbesondere C₁-C₆-Alkyl, C₇-C₁₂-Aralkyl oder C₆-C₁₄-Aryl, insbesondere Phenol, oder für C₃-C₈-Cycloalkyl (jeweils wie vorstehend für die Reste R¹ und R² genannt) stehen und wobei mindestens einer der Reste X und Y nicht Wasserstoff bedeutet.

Die Reste X und R¹ oder Y und R¹ können gemeinsam auch einen 5- bis 8-gliedrigen gesättigten oder ungesättigten und gegebenenfalls substituierten Ring, beispielsweise einen Cyclopentyl- einen Cyclohexyl- oder einen Cyclooctyl-Rest darstellen.

Die Reste R³ und R⁴, R⁵ und R⁶ sowie R⁹ und R¹⁰ können unabhängig voneinander jeweils gemeinsam auch für -(CH₂)ₘ- stehen, wobei m eine ganze Zahl von 4 bis 7, insbesondere 4 oder 5 bedeutet Dabei kann eine CH₂-Gruppe durch O oder NR⁸ ersetzt sein,

Die Reste R¹ und R⁵ können auch gemeinsam für -(CH₂)ₙ,- stehen, wobei n einer ganzen Zahl von 2 bis 6 entspricht.

Der Rest R⁷ steht vorzugsweise für Wasserstoff oder für geradkettiges oder verzweigtes C₁-C₁₂-Alkyl oder C₃-C₈-Cyclooalkyl, besonders bevorzugt für Methyl, Ethyl, 1-Methylethyl, 1,1,Dimethylethyl, Hexyl, Cyclohexyl oder Dodecyl. Er kann auch gemeinsam mit R¹ für-(CH₂)ₙ- stehen, wobei n einer ganzen Zahl von 2 bis 6 entspricht.

Für den Fall der Herstellung optisch aktiver 2-Amino-, 2-Chlor-, 2-Hydroxy- und 2-Alkoxy-1-alkanole in Gegenwart von Palladium und Rhenium oder Platin und Rhenium enthaltenden Katalysatoren sind 2-Amino-, 2-Chlor-, 2-Hydroxy und 2-AlkoxyCarbonsäuren und ihre Säurederivate aus der Gruppe der erfindungsgemäß umzusetzenden Verbindungen ausgenommen.

Das erfindungsgemäße Verfahren eignet sich auch zur Umsetzung optisch aktiver Dicarbonsäuren oder deren Säurederivate, insbesondere solcher der Formel (II) 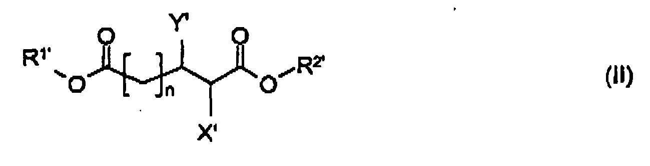 wobei
- X', Y':: unabhängig voneinander Wasserstoff, Chlor, NR^{5'}R^{6'} oder OR^{7'}, geradkettiges oder verzweigtes C₁-C₁₀-Alkyl oder C₆-C₁₀-Aryl mit der Maßgabe dass mindestens einer der Reste X' oder Y' nicht Wasserstoff ist,
- R^{1'}, R^{2'}:: unabhängig voneinander Wasserstoff, geradkettiges oder verzweigtes C₁-C₁₂-Alkyl oder G₃-C₈-Cycloalkyl und
- n: eine ganze Zahl von 0 bis 8 bedeutet
- R^{5'}, R^{6'}:: unabhängig voneinander jeweils Wasserstoff, geradkettiges und verzweigtes C₁-C₁₂-Alkyl, C₇-C₁₂-Aralkyl, C₆-C₁₄-Aryl, C₃-C₈-Cycloalkyl oder C₃-C₈-Cycloalkyl, in dem eine CH₂-Gruppe durch O oder NR⁵_{,} ersetzt ist und gemeinsam auch -CH₂)ₘ-, wobei m eine ganze Zahl von 4 bis 7 bedeutet,
- R^{7'}:: Wasserstoff, geradkettiges oder verzweigtes C₁-C₁₂-Alkyl oder C₃-C₈-Cycloalkyl und
- R^{8'}:: Wasserstoff, geradkettiges oder verzweigtes C₁-C₁₂-Alkyl, C₇-C₁₂-Aralkyl oder C₆-C₁₄-Aryl
bedeutet, zu den entsprechenden optisch aktiven Hydroxycarbonsäuren oder deren Säurederivaten oder, im Fall der Hydrierung beider Carbonsäurefunktionen, zu den entsprechenden optisch aktiven substituierten Diolen. So lassen sich beispielsweise optisch aktive Hydroxydicarbonsäuren auch zu den entsprechend optisch aktiven Triolen hydrieren.

R^{1'} und R^{2'} können beispielhaft und unabhängig voneinander folgende Bedeutungen annehmen: Wasserstoff, geradkettiges oder verzweigtes C₁-C₁₂Alkyl (wie vorstehend für Rest R¹ in Formel I genannt) oder C₃-C₈-Cycloalkyl wie z. B. Cyclopropyl, Cyclobutyl, Cyclopentyl, Cyclohexyl, Cycloheptyl und Cyclooctyl.

Anstelle der Carbonsäureester können auch die Säureanhydride als Carbonsäurederivate eingesetzt werden.

Die Reste X' und Y' stehen unabhängig voneinander für Wasserstoff, Chlor, NR^{5"}R^{8'} oder OR^{7'}, wobei R^{5'} und R^{6'} unabhängig voneinander jeweils für Wasserstoff, geradkettiges und verzweigtes C₁-C₁₂-Alkyl, insbesondere C₁-C₆-Alkyl, C₇-C₁₂-Aralkyl oder C₆-C₁₄-Aryl, insbesondere Phenyl, oder für C₃-C₈-Cycloalkyl (jeweils wie vorstehend für die Reste R¹ und R² in Formeln I genannt) stehen.

Die Reste R^{5'} und R^{6'} können unabhängig voneinander jeweils gemeinsam auch für -(CH₂)ₘ- stehen, wobei m eine ganze Zahl von 4 bis 7, insbesondere 4 oder 5 bedeutet Dabei kann eine CH₂-Gruppe durch O oder NR^{8'} ersetzt sein.

Der Rest R^{7'} steht vorzugsweise für Wasserstoff oder für geradkettiges oder verzweigtes C₁-C₁₂-Alkyl oder C₃-C₈-Cycloalkyl, besonders bevorzugt für Methyl, Ethyl, 1-Methylethyl, 1,1-Dimethylethyl, Hexyl, Cyclohexyl oder Dodecyl.

Die durch das erfindungsgemäße Verfahren durch Hydrierung optisch aktiver Dicarbonsäuren, beispielsweise solcher der Formel II, erhältlichen optisch aktiven Hydroxycarbonsäuren oder Diole können unter geeigneten Bedingungen auch durch intramolekulare Cyclisierung optisch aktive Cyclisierungsprodukte, beispielsweise Lactone, Lactame oder cyclische Ether bilden. Bevorzugte Cyclisierungsprodukte stellen die Lactone und cyclische Ether dar, deren Herstellung in optisch aktiver Form durch Hydrierung optisch aktiver Dicarbonsäuren und anschließender Cyclisierung auch ein Gegenstand dieser Erfindung ist. Bevorzugte, in erfindungsgemäßer Weise ausgehend von optisch aktiven Dicarbonsäuren der Formel II zugängliche optisch aktive Lactone sind beispielsweise solche der Formel III oder IV 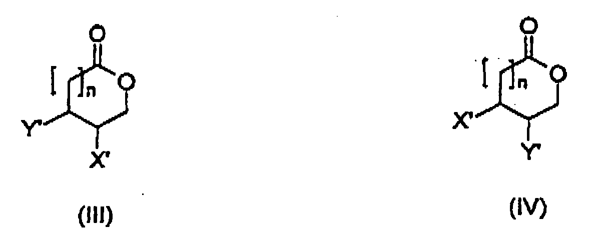 wobei die Reste X', Y' und n die für Formel II angegebenen Bedeutungen besitzen.

Bevorzugte, in erfindungsgemäßer Weise ausgehend von optisch aktiven Dicarbonsäuren der Formel II in optisch aktiver Form zugängliche cyclische Ether sind beispielsweise solche der Formel V oder VI 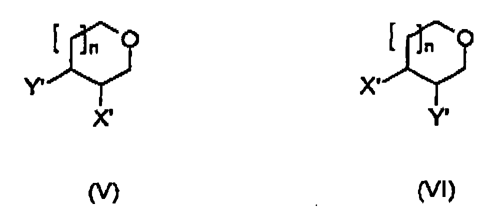 wobei die Reste X', Y' und n die für Formel II angegebenen Bedeutungen besitzen.

Auf diese Weise sind durch das erfindungsgemäße Verfahren beispielsweise folgende Lactone in optisch aktiver Form zugänglich: 2-Hydroxy-γ-butyrolacton, 3-Hydroxy-γbutyrolacton, 2-Chlor-γ-butyrolacton, 3-chlor-γ-butyrolacton, 2-Amino-γ-butyrolacton, 3-Amino-γ-butyrolacton, 2-Methyl-γ-butyrolacton, 3-Methyl-γ-butyrolacton; 3-Hydroxy-δvalerolacton, 4-Hydroxy-δ-valerolacton.

Darunter ist 3-Hydroxy-y-butyrolacton in optisch aktiver Form im Rahmen des erfindungsgemäßen Hersteliverfahrens besonders bevorzugt.

Als durch das erfindungsgemäße Verfahren in optisch aktiver Form zugängliche cyclische Ether seien beispielhaft genannt 2-Hydroxy-Tetrahydrofuran, 2-Methyl-Tetrahydrofuran und 2-Amino-Tetrahydrofuran.

Eine bevorzugte Ausführungsform des erfindungsgemäßen Verfahrens betrifft die Herstellung optisch aktiver 3-Hydroxy-, 3-Alkoxy-, 3-Amino-, 3-Alkyl-, 3-Aryl- oder 3-Chlor-1-Alkanole ausgehend von den entsprechend substituierten optisch aktiven 3-Hydroxy-, 3-Alkoxy-, 3-Amino-, 3-Alkyl-, 3-Aryl- oder 3-Chlor-Monocarbonsäuren oder deren Säurederivaten unter Verwendung von Palladium und Rhenium oder Platin und Rhenium enthaltenden Katalysatoren.

So sind in erfindungsgemäßer Weise unter Verwendung von Palladium und Rhenium oder Platin und Rhenium enthaltenden Katalysatoren 3-Hydroxy-., 3-Alkoxy-, 3-Amino-, 3-Alkyl-, 3-Aryh oder 3-Chlor-Alkohole bzw. Diole beispielsweise zugänglich durch Umsetzung optisch aktiver Carbonsäuren oder deren Säurederivate der Formel VII, 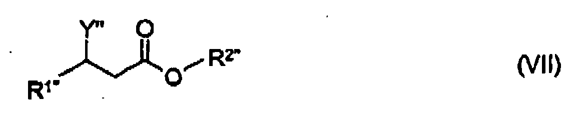 in der die Reste folgende Bedeutung haben:
- R^{1"}:: Geradkettiges und verzweigtes C₁-C₁₂-Alkyl, C₇-C₁₂-Aralkyl oder C₆-C₁₄-Aryl, wobei die genannten Reste durch NR^{3"}R^{4"}, OH, COOR^{2"'} und/oder weitere, unter den Reaktionsbedingungen stabile Gruppen substituiert sein können,
- R^{2"}, R^{2"'}:: unabhängig voneinander jeweils Wasserstoff, geradkettiges oder verzweigtes C₁-C₁₂-Alkyl oder C₃-C₈-Cycloalkyl,
- Y":: Chlor, NR^{5"}R^{6"} oder OR^{7"}, geradkettiges oder verzweigtes C₁-C₁₀-Alkyl oder C₆-C₁₄-Aryl,
- R^{3"}, R^{4"}, R^{5"} und R^{6"}:: Unabhängig voneinander jeweils Wasserstoff, geradkettiges und verzweigtes C₁-C₁₂-Alkyl, C₇-C₁₂-Aralkyl, C₆-C₁₄-Aryl, C₃-C₈-Cycloalkyl oder G₃-C₈-Cycloalkyl, in dem eine CH₂-Gruppe durch O oder NR^{8"} ersetzt ist.
- R^{3"} und R^{4"} sowie R^{5"} und R^{6"}:: Unabhängig voneinander jeweils gemeinsam auch -(CH₂)ₘ-, wobei m eine ganze. Zahl von 4 bis 7 bedeutet,
- R^{1"} und R^{5"}:: Gemeinsam auch -(CH₂)ₙ-, wobei n einer ganzen Zahl von 2 bis 6 entspricht,
- R^{7"}:: Wasserstoff, geradkettiges oder verzweigtes C₁-C₁₂-Alkyl oder C₃-C₈-Cycloalkyl,
- R^{1"} und R^{7"}:: Gemeinsam auch -(CH₂)ₙ-, wobei n einer ganzen Zahl von 2 bis 6 entspricht und
- R^{8"}:: Wasserstoff, geradkettiges oder verzweigtes C₁-C₁₂-Alkyl, C₇-C₁₂-Aralkyl oder C₆-C₁₀-Aryl.

Beispiele für die genannten Reste entsprechen den for die Reste in Formel I genannten.

Eine weitere bevorzugte Ausführungsfonn des erfindungsgemäßen Verfahrens betrifft die Herstellung optisch aktiver 2-Alkyl- und 2-Aryl-1-Alkanole ausgehend von den entsprechend substituierten optisch aktiven 2-Alkyl- bzw. 2-Arylcarbonsäuren oder deren Säurederivaten unter Verwendung von Palladium und Rhenium oder Platin und Rhenium enthaltenden Katalysatoren. Dabei kommt den Alkyl- bzw. Aryl-Subsütuenten die für die Reste X bzw. Y in Formel I angegeben Bedeutung zu.

Als bevorzugte, durch das erfindungsgemäße Verfahren in optisch aktiver Form zugängliche Verbindungen seien beispielhaft genannt:
1,2- und 1,3-Aminoalkohole wie beispielsweise: α-Alaninol, sowie jeweils in der α- oder β-Form; Leucinol, Isoserinol, Valinol, Isoleucinol, Serinol, Threoninol, Lysinol, Phenylalaninol, Tyrosinol, Prolinol sowie die aus den Aminosäuren Ornithin, Citrullein, Asparagin, Asparaginsäure, Glutamin und Glutaminsäure erhältlichen Alkohole durch Umsetzung der entsprechenden optisch aktiven α- oder β-Aminosäuren oder deren Säurederivate,
1,2- und 1,3-Alkandiole wie beispielsweise: 1,2-Propandiol, 1,2-Butandiol, 1,2-Pentandiol, 1,3-pentandiol durch Umsetzung der entsprechenden optisch aktiven α-oder β-Hydroxycarbonsäuren oder deren Säurederivate,
1,2- und 1,3-Chloralkohole wie beispielsweise 2-Chlorpropanol durch Umsetzung der entsprechenden optisch aktiven α- oder β-Chlorcarbonsäuren α- oder β-Chlordicarbonsäuren oder deren Säurederivate,
1,2- und 1,3-Alkylalkohole wie beispielsweise 2-Methyl-1-butanol, 2,3-Dimethylbutan-1,4-diol, 2-Methylbutan-1,4-diol durch Umsetzung der entsprechenden optisch aktiven α- oder β-Alkyl-Carbonsäuren oder deren Säurederivate,
Triole wie beispielsweise 1,2,4-Butantriol, 1,2,5-Pentantrlol, 1 ,2,6-Hexantriol durch Umsetzung der entsprechenden optisch aktiven α- oder β-Hydroxy-Hydroxydicarbonsäuren und Dihydroxycarbonsäuren oder deren Säurederivate wie z.B. 3,4-Dihydroxybuttersäure durch Umsetzung der entsprechenden optisch aktiven Dicarbonsäuren.

Zur Durchführung des erfindungsgemäßen Hydrierverfahrens eignen sich solche Katalysatoren, deren Aktivkomponente aus Re besteht, wie z. B. von Re-Schwämmen oder Rhenium auf einem geeigneten Träger wie z.B. Re auf Aktivkohle, TiO₂, ZrO₂ oder High Surface Activated Graphite oder solche Katalysatoren, deren Aktivkomponente Rhenium und mindestens ein weiteres Element ausgewählt aus der Gruppe der Elemente Pd, Pt, Rh, Ir, Cu, Ag, Au, Cr, Mo, W, Co und Ni enthält.

Im Rahmen des erfindungsgemäßen Verfahrens bevorzugte Katalysatoren sind solche, deren Aktivkomponente Rhenium und mindestens ein weiteres Element ausgewählt aus der Gruppe der Elemente : Rh, Ir, Cu, Ag, Au, Cr, Mo, W, Co und Ni enthält.

Besonders bevorzugt sind solche Katalysatoren, deren Aktivkomponente Rhenium und mindestens ein weiteres Element ausgewählt aus der Gruppe der Elemente: Pd, Pt, Rh, Ir enthalten. Insbesondere bevorzugt sind solche Katalysatoren, deren Aktivkomponente aus Re und einem weiteren Element ausgewählt aus der Gruppe der Elemente: Pd, Pt, Rh, Ir besteht. Darunter wiederum bevorzugt sind solche Katalysatoren deren Aktivkomponente aus Re und einem weiteren Element ausgewählt aus der Gruppe der Elemente: Rh, Ir besteht.

Die erfindungsgemäßen Katalysatoren können mit gutem Erfolg als Voll- oder als Trägerkatalysator eingesetzt werden. Trägerkatalysatoren zeichnen sich dadurch aus, dass die gewählte Aktivkomponente auf die Oberfläche eines geeigneten Trägers aufgebracht ist Zur Durchführung des erfindungsgemäßen Hydrierverfahrens besonders bevorzugt sind Trägerkatalysatoren, die eine hohe Oberfläche besitzen und daher geringere Mengen der aktiven Metalle benötigen.

Die Vollkatalysatoren sind beispielsweise herstellbar durch Reduktion einer Aufschlämmung und/oder Lösung im wässrigen oder organischen Medium von Rhenium und der weiteren, erfindungsgemäßen Aktivkomponente in metallischer Form oder in Form von Verbindungen, wie beispielweise Oxiden, Oxidhydraten, Carbonaten, Nitraten, Carboxylaten, Sulfaten, Phosphaten, Halogeniden, Wemerschen Komplexen, metallorganischen Komplexen oder Chelatkomplexen oder Mischungen davon.

Beim Einsatz der Katalysatoren als Trägerkatalysator sind Träger wie Kohlen, Ruße, Graphite, High Surface Activated Graphite (HSAG), SiO₂, Al₂O₃, TiO₂, ZrO₂, SiC, Tonerden, Silikate, Montmorillonite, Zeolithe oder Mischungen hiervon bevorzugt. Besonders bevorzugt für den Einsatz als Trägermaterialien sind Kohlen, Graphite, HSAG, TiO₂ und ZrO₂.

Bei den kohlenstoffbasierten Trägem (Aktivkohle, Graphite, Ruße, HSAG) ist es erfindungsgemäß vorteilhaft, das Trägermaterial oxidativ mit übliches Oxidationsmitteln wie z. B. HNO₃, H₂O₂, O₂, Luft, O₃, Ammoniumpersulfat, Natriumhypochlorid, hypochloriger Säure, Perchlorsäure, Nitratsalzen und/oder mit Säuren wie HNO₃, N₃PO₄, HCl oder HCOOH zu behandeln. Besonders bevorzugt ist die Vorbehandlung mit HNO₃, H₃PO₄ oder HCOOH. Der Träger kann dabei vor oder während der Aufbringung der Metalle behandelt werden. Durch die Vorbehandlung lassen sich Aktivität und Selektivität der Trägerkatalysatoren in der erfindungsgemäßen Hydrierung verbessern.

Die erfindungsgemäßen Trägerkatalysatoren enthalten üblicherweise etwa 0,01 bis 50 Gew.-% Rhenium in metallischer Form oder in Form von Verbindungen und 0,01 bis 30 Gew.-% mindestens eines weiteren Elements ausgewählt aus der Gruppe der Elemente Pd, Pt, Rh, Ir, Cu, Ag, Au, Cr, Mo, W, Co und Ni in der metallischen Form oder in Form einer Verbindung oder Gemischen hiervon. Die Gew: %-Angaben sind dabei jeweils bezogen auf das Gesamtgewicht der Katalysatoren und berechnet in metallischer Form.

Der Anteil Rhenium als Metall gerechnet beträgt vorzugsweise etwa 0.1 bis 30 Gew.-%, besonders bevorzugt etwa 1 bis 20 Gew.-%, bezogen auf das Gesamtgewicht des Trägerkatalysators.

Der Anteil des mindestens einen weiteren Elements ausgewählt aus der Gruppe der Elemente Pd, Pt, Rh, Ir, Cu, Ag, Au, Cr, Mo, W, Co und Ni ist bevorzugt etwa 0,1 bis 20 Gew.-% und besonders bevorzugt etwa 1 bis 10 Gew.-%, bezogen auf das Gesamtgewicht des fertigen Trägerkatalysators.

Als Rheniumkomportente wind üblicherweise Re₂O₇, ReO₂, ReCl₃, ReCl₅, HReO₄, Re(CO)₅Cl, Re(CO)₅Br, oder Re₂(CO)₁₀ oder Rheniumkomplexe eingesetzt. Besonders bevorzugt sind Re₂O₇ und HReO₄.

Die neben Rhenium weiteren Elemente ausgewählt aus der Gruppe der Elemente Pd, Pt, Rh, Ir, Cu, Ag, Au, Cr, Mo, W, Co und Ni werden üblicherweise in Form von Metall, Oxiden, Oxidhydraten, Carbonate, Nitraten, Carboxylaten, Sulfaten. Phosphaten, Wemerschen Komplexen, Chelatkomplexen oder Halogeniden auf das Trägermaterial aufgebracht Bevorzugt sind dabei Verbindungen von Pt, Pd, Rh oder Ir. Insbesondere bevorzugt sind Ir und Rh in Form der Nitrate.

Die Aufbringung der Aktivkomponenten kann in einem oder mehreren Schritten durch Imprägnierung mit einer wässrigen oder alkoholischen Lösung der jeweiligen gelösten Salze oder Oxide oder von gelösten oxidischen oder metallischen Kolloiden hergestellt werden, oder durch Gleichgewichtsadsorption in einem oder mehreren Schritten der in wässriger oder alkoholischer Lösung gelösten Salze oder Oxide oder von gelösten oxidischen oder metallischen Kolloiden. Zwischen einzelnen Gleichgewichtsadsorptions- bzw. Imprägnierschritten kann jeweils ein Trocknungsschritt zur Entfernung des Lösungsmittels und wahlweise ein Kalzinierschritt oder Reduktionsschritt durchgeführt werden.

Die Trocknung wird dabei vorteilhaft jeweils bei Temperaturen von etwa 25 bis etwa 350°C durchgeführt, bevorzugt von etwa 40 bis etwa 280°C und besonders bevorzugt von etwa 50 bis etwa 150°C.

Wahlweise kann eine Kalzinierung nach jedem Aufbringungs- oder Trocknungsschritt bei Temperaturen im Bereich von etwa 100 bis 800°C erfolgen, bevorzugt bei etwa 200 bis etwa 600°C und besonders bevorzugt bei etwa 300 bis etwa 500°C.

Wahlweise kann nach jedem Aufbringungsschritt eine Reduktion durchgeführt werden.

In einer besonderen Ausführungsform der Herstellung der erfindungsgemäß einsetzbaren Trägerkatalysatoren wird in einem ersten Imprägnierschritt ein Metall ausgewählt aus der Gruppe der Elemente Pd, Pt, Rh, Ir, Cu, Ag, Au, Cr, Mo, W, Co und Ni aus den jeweiligen Oxiden, Oxidhydraten, Carbonate, Nitraten, Carboxylaten, Sulfaten, Phosphaten, Wemerschen Komplexen, Chelatkomplexen oder Halogeniden auf den Träger aufgebracht, dann erfolgt ein Trocknungsschritt und wahlweise ein Kalzinierschritt und wahlweise ein Reduktionsschritt. Danach erfolgt wahlweise eine weitere Imprägnierung mit einem oder mehreren Metallen ausgewählt aus der Gruppe der Elemente Pd, Pt, Rh, Ir, Cu, Ag, Au, Cr, Mo, W, Co und Ni aus den jeweiligen Oxiden, Oxidhydraten, Carbonate, Nitraten, Carboxylaten, Sulfaten, Phosphaten, Wemerschen Komplexen, Chelatkomplexen oder Halogeniden mit folgender Trockunung und wahlweiser Kalzinierung. Im letzten Herstellungsschritt wird dann Rhenium in Form von Re₂O₇, ReO₂, ReCl₃, ReCl₅, HReO₄, Re(CO)₅Cl, Re(CO)₅Br, oder Re₂CO)₁₀ auf den Träger aufgebracht Abschließend erfolgt ein weiterer Trocknungs- und wahlweise ein Kalzinierungsschritt.

Eine weitere Möglichkeit zur Herstellung der erfindungsgemäßen Trägerkatalysatoren besteht in der stromlosen Abscheidung von Rhenium und mindestens einer weiteren metallischen Komponent ausgewählt aus der Gruppe der Elemente Pd, Pt, Rh, Ir, Cu, Ag, Au, Cr, Mo, W, Co und Ni aus den jeweiligen Oxiden, Oxidhydraten, Carbonaten, Nitraten, Carboxylaten, Sulfaten, Phosphaten, Wemerschen Komplexen, Chelatkomplexen oder Halogeniden auf das Trägermaterial. Die stromlose Abscheidung erfolgt vorteilhafterweise in wässriger oder alkoholischer Aufschlämmung des Trägermaterials und den jeweiligen Metallverbindungen durch Zugabe von Reduktionsmitten wie z. B. Alkoholen oder Natriumhypophosphit, Ameisensäure, Alkali-Formiaten, insbesondere Natriumformiat. Besonders bevorzugt sind Ethanol und NaH₂PO₂.

Nach der Abscheidung führt man vorteilhaft einen Trocknungsschritt bei Temperaturen im Bereich von etwa 25 bis etwa 350°C, bevorzugt von etwa 40 bis etwa 280°C und besonders bevorzugt von etwa 50 bis etwa 150°C durch.

Wahlweise kann eine Kalzinierung nach der Abscheidung bei Temperaturen im Bereich von etwa 100 bis etwa 800°C, bevorzugt von etwa 200 bis etwa 600°C und besonders bevorzugt von etwa 300 bis etwa 500°C erfolgen.

Die erfingdungsgemäß eingesetzten Katalysatoren werden üblicherweise vor Verwendung aktiviert. Bei den durch stromlose Abscheidung hergestellten Katalysatoren kann wahlweise auf diesen Aktivierungsschritt verzichtet werden. Bevorzugt wird zur Aktivierung Wasserstoff oder ein Gemisch aus Wasserstoff und einem Inertgas, üblicherweise eine Mischung aus H₂ und N₂. Die Aktivierung wird bei Temperaturen von etwa 100 bis etwa 500°C. bevorzugt von etwa 140 bis etwa 400°C und besonders bevorzugt von etwa 180 bis etwa 330°C durchgeführt. Dabei wird bei Drucken von etwa 1 bis etwa 300 bar aktiviert, bevorzugt bei etwa 5 bis etwa 200 bar und besonders bevorzugt bei etwa 10 bis etwa 100 bar.

Die erfindungsgemäß einsetzbaren Katalysatoren haben üblicherweise eine Spezifische Oberfläche von etwa 5 bis 3000 m²/g, vorzugsweise von etwa 10 bis etwa 1500 m²/g,

Die erfindungsgemäße Hydrierreaktion erfolgt üblicherweise in Gegenwart von Wasserstoff bei Temperaturen im Bereich von etwa 10 bis etwa 300°C, bevorzugt von etwa 30 bis etwa 180°C und besonders bevorzugt von etwa 50 bis 130°C. Dabei wird in der Regel ein Druck von etwa 1 bis etwa 350 bar, bevorzugt von etwa 10 bis etwa 300 bar und besonders bevorzugt von etwa 100 bis etwa 300 bar angewendet.

Für den Fall der erfindungsgemäßen Hydrierung von optisch aktiven Dicarbonsäuren zu den entsprechenden optisch aktiven Diolen wählt man bevorzugt einen Druck von etwa 150 bis etwa 250°C, besonders bevorzugt von etwa 180 bis etwa 250°C und ganz besonders bevorzugt von etwa 200 bis etwa 250°C.

In einer bevorzugten Ausführungsform des erfindungsgemäßen Verfahrens, insbesondere zur Hydrierung von Amino-substituierten Substraten, werden die oben beschriebenen optisch aktiven Ausgangsstoffe in Gegenwart einer organischen oder anorganischen Säure hydriert. In der Regel beträgt der Zusatz an Säure 0,5 bis 1,5 Äquivalente, besonders bevorzugt 1 bis 1,3 Äquivalente, bezogen auf 1 Äquivalent der in den Ausgangsstoffen gegebenenfalls vorhandenen basischen Gruppen. Als organische Säuren kommen beispielsweise Essigsäure, Propionsäure und Adipinsäure in Betracht Bevorzugt ist der Zusatz anorganischer Säuren, insbesondere Schwefelsäure, Salzsäure und Phosphorsäure. Die Säuren können beispielsweise als solche, in Form wässriger Lösungen oder in Form ihrer separat hergestellten Salze mit den zu hydrierenden Ausgangsstoffen, z.B. als Sulfate, Hydrogensulfate, Hydrochloride, Phosphate, Mono- oder Dihydrogenphosphate eingesetzt werden.

Die umzusetzende optisch aktive Carbonsäure bzw. Dicarbonsäure kann mit gutem Erfolg in Substanz oder in Form einer wässrigen oder organischen Lösung eingesetzt werden. Die Hydrierung kann in Suspension oder in kontinuierlicher Fahrweise im Festbettreaktor in der Flüssig- oder Gasphase durchgeführt werden.

Im Falle diskontinuierlicher Reaktionsführung kann man bezogen auf 1 Mol eingesetzter optisch aktiver Ausgangsverbindung z.B. 0,1 bis 50 g der erfindungsgemäß einzusetzenden Vollkatalysatoren oder auch etwa 0,1 bis 50 g erfindungsgemäß einzusetzender Trägerkatalysatoren verwenden.

Bei kontinuierlicher Verfahrensführung wählt man das Mengenverhältnis von Katalysator zur umzusetzende Ausgangsverbindung vorteilhaft so, dass sich eine Katalysator belastung im Bereich von etwa 0,005 bis etwa 2 kg/Iₖₐₜh, vorzugsweise von etwa 0,02 bis etwa 0,5 kg/Iₖₐₜh.

Als Lösungsmittel für die Reaktion eignen sich beispielsweise die Hydrierprodukte selbst, Wasser, Alkohole wie z. B. Methanol, Ethanol, Propanol, Butanol, Ether wie z.B. THF oder Ethylenglykolether. Bevorzugt werden Wasser oder Methanol oder Gemische derselben als Lösungsmittel eingesetzt.

Die Hydrierung kann in der Gas- oder Flüssigphase ein- oder mehrstufig durchgeführt werden. In der Flüssigphase ist die Suspensions- oder Festbettfahrweise möglich. Zur Durchführung des erfindungsgemäßen Verfahrens eignen sich alle dem Fachmann als zur Durchführung von Hydrierungen geeignet bekannten Reaktoren wie beispielsweise: Rührkessel, Festbettreaktoren, Schachtreaktoren, Rohrbündelreaktoren, Blasensäulen oder Wirbelbettreaktoren.

Die Reaktion ist üblicherweise beendet, wenn kein Wasserstoff mehr aufgenommen wird. Üblicherweise beträgt die Reaktionszeit etwa 1 bis etwa 72 h.

Die Isolierung und nötigenfalls Trennung der anfallenden Reaktionsprodukte kann prinzipiell nach allen üblichen und dem Fachmann an sich bekannten Verfahren durchgeführt werden. Insbesondere eignen sich dazu extraktive und destillative Verfahren sowie die Aufreinigung bzw. Isolierung durch Kristallisation.

Die eingesetzten bzw. erhaltene optisch aktive Edukte bzw. Produkte können mittels aller dem Fachmann bekannten Methoden auf ihren Enantiomerenreinheit hin untersucht werden. Besonders geeignet sind dazu insbesondere chromatographische Verfahren, speziell gaschromatographische Verfahren oder Verfahren der Hochleistungsflüssigkeitschromatographie (HPLC). Ein geeignetes Maß zur Bestimmung der Enantiomerenreinheit der Edukte bzw. Produkte stellt der Enantiomerenüberschuss (ee) dar.

Das erfindungsgemäße Verfahren zeichnet sich dadurch aus, dass die Racemisierung stereogener Zentren der in optisch aktiver Form als Ausgangsverbindungen eingesetzten substituierten Mono- oder Dicarbonsäuren bei der Hydrierung weitgehend unterdrückt wird. Demgemäss entspricht im Rahmen des erfindungsgemäßen Verfahrens der Enantiomerenüberschuss der erhaltenen Produkte üblicherweise weitgehend dem der eingesetzten Edukte. Bevorzugt wählt man die Reaktionbedingungen so, dass der Enantiomerenüberschuss des gewünschten Produktes zu mindestens 90 %, besonders bevorzugt zu mindestens 95 %, ganz besonders bevorzugt zu mindestens 98 % dem der eingesetzten Ausgangsverbindung entspricht

Ein Vorteil des erfindungsgemäßen Verfahrens besteht darin, dass die als störende Nebenreaktion bei derartigen Umsetzungen bekannte Decarbonylierung unter Freisetzung von Kohlenmonoxid und dessen anschließender Reduktion zum Methan oder anderen niederen Alkanen weitgehend unterdrückt wird. Dies führt zu erheblichen sicherheitstechnischen Vorteilen.

Die folgenden Beispiele dienen der Erläuterung des erfindungsgemäßen Verfahrens, ohne es jedoch in irgendeiner Weise zu beschränken:
Allgemeine Vorschrift zur Aktivierung der Trägermaterialien durch Behandlung mit einer Säure:
   100 g des gewählten Trägermaterials wurden mit 200 ml der gewählten Säure und 400 ml Wasser für 45 min unter Rühren auf 104°C erhitzt. Nach Abfiltrieren und Waschen mit Wasser wurde das aktivierte Trägermaterial bei 80°C im Umluftofen getrocknet, Bei der Verwendung von Formkörpern kann die Aktivierung auch im Rotationsverdampfer oder in einem mit der Aktivierungslösung durchströmten Festbettreaktor durchgeführt werden, um die mechanische Zerstörung des Trägers zu minimieren.

### Herstellvorschrift Katalysators 1:

In einer 2 l Rührapparatur wurden 25 g mit HCOOH vorbehandelter Timrex^{®} HSAG 100 (Fa. Timcal), Natriumhypophosphit 4,9 g Re₂O₇ und 5,4 g Pd(NO₃)₂ in 1400 ml Wasser vorgelegt und 30 min. bei Raumtemperatur und danach bei 80°C gerührt. Anschließend wurde über eine Nutsche abgesaugt, gewaschen und getrocknet.

### Herstellvorschrift Katalysator 2:

Mit einer Lösung von 14 g Rh(NO₃)₃ und 9,8 g Re₂O₇ in 72 ml Wasser wurden 100 g von HCOOH-aktiviertem Timrex^{®} HSAG 100 (Fa. Timcal) imprägniert. Nach 16-stündiger Trocknung wurde erneut mit einer Lösung von 14g Rh(NO₂)₃ und 9,8 g Re₂O₇ in 72 ml Wasser imprägniert und erneut getrocknet Abschließend wurde bei 400°C im Drehrohr kalziniert.

### Herstellvorschrift Katalysator 3:

Es wurden 2 g Pt(NO₃)₂ und 3 g Re₂O₇ in Wasser gelöst und auf 18 ml Wasser ergänzt. Damit wurden 25 g HCOOH-aktivierter Timrex^{®} HSAG 100 imprägniert, getrocknet und kalziniert.

### Hersteilvorschrift Katalysator 4:

Mit einer Lösung von 14 g Rh(NO₃)₃ und 9,8 g Re₂O₇ in 72 ml Wasser wurden 100 g von HNO₃-aktiviertem Timrex^{®} HSAG 100 (Fa. Timcal) imprägniert Nach 16-stündiger Trocknung wurde erneut mit einer Lösung von 14 g Rh(NO₃)₃ und 9,8 g Re₂O₇ in 72 ml Wasser imprägniert und erneut getrocknet. Abschließend wurde bei 400°C im Drehrohr kalziniert.

### Herstellvorschrift Katalysator 5:

Mit einer Lösung von 14 g Rh(NO₃)₃ und 9,8 g Re₂O₇ in 72 ml Wasser wurden 100 g von HNO₃-aktiviertem Aktivkohtesträgnen (3 mm) imprägniert. Nach 16-stündiger Trocknung wurde erneut mit einer Lösung von 14 g Rh(NO₃)₃ und 9,8 g Re₂O₇ in 72 ml Wasser imprägniert und erneut getrocknet. Abschließend wurde bei 400°C im Drehrohr kalziniert

### Herstelfvorschrift Katalysator 6:

Es wurden 4,8 g IrCₗxH₂O und 4,9 g Re₂O₇ in Wasser gelöst und auf 18 ml Wasser ergänzt. Damit wurden 25 g HCOOH-aktivierter Timrex^{®} HSAG 100 imprägniert, getrocknet und kalziniert.

### Herstellvorschrift Katalysator 7:

Es wurden 2,7 g Pd(NO₃)₂ und 2,4 g Re₂O₇ in Wasser gelöst und auf 18 ml Wasser ergänzt. Damit wurden 25 g HCOOH-aktivierter Timrex^{®} HSAG 100 imprägniert. Nach 16-stündiger Trocknung wurde erneut mit einer Lösung von 2,7 g Pd(NO₃)₂ und 2,4 g Re₂O₇ in 18 ml Wasser imprägniert und erneut getrocknet. Abschließend wurde bei 400°C im Drehrohr kalziniert

### Herstellvorschrift Katalysator 8:

Es wurden 0,6 g Pd(NO₃)₂ in Wasser gelöst und auf 18 ml Gesamtlösung ergänzt. Damit wurden 25 g HCOOH-aktivierter Timrex^{®} HSAG 100 entsprechend seiner Wasseraufnahme imprägniert. Nach 16-stündiger Trocknung und Kalzinierung bei 400°C im Drehrohr erfolgte eine zweite Imprägnierung, zu der 2,9 g Re₂O₇ in Wasser gelöst und auf 18 ml Gesamtlösung ergänzt wurden. Damit wurde das Material ein zweites mal entsprechend seiner Wasseraufnahme imprägniert und erneut 16 Stunden getrocknet.

### Herstellvorschrift Katalysator 9:

Es wurden 0,5 g Pt(NO₃)₂ in Wasser gelöst und auf 18 ml Gesamtlösung ergänzt. Damit wurden 25 g HCOOH-aktnrierter Timrex^{®} HSAG 100 entsprechend seiner Wasseraufnahme imprägniert Nach 16-stündiger Trocknung und Kalzinierung bei 400°C im Drehrohr erfolgte eine zweite Imprägnierung, zu der 3,7 g Re₂O₇ in Wasser gelöst und auf 18 ml Gesamtlösung ergänzt wurden. Damit wurde das Material ein zweites mal entsprechend seiner Wasseraufnahme imprägniert und erneut 16 Stunden getrocknet

### Herstellvorschrift Katalysator 10:

Es wurden 1,5 g Rh(NO₃)₃ in Wasser gelöst und auf 18 ml Gesamtlösung ergänzt. Damit wurden 25 g HCOOH-aktivierter Timrex^{®} HSAG 100 entsprechend seiner Wasseraufnahme imprägniert. Nach 16-ständiger Trocknung und Kalzinierung bei 400°C im Drehrohr erfolgte eine zweite Imprägnierung, zu der 2 g Re₂O₇ in Wasser gelöst und auf 18 ml Gesamtlösung ergänzt wurden. Damit wurde das Material ein zweites mal entsprechend seiner Wasseraufnahme imprägniert und erneut 16 Stunden getrocknet

### Herstellvorschrift Katalysator 11:

Es wurden 0,7 g Pd(NO₃)₂ in Wasser gelöst und auf 18 ml Gesamtlösung ergänzt. Damit wurden 25 g HNO₃-aktivierter Timrex^{®} HSAG 100 entsprechend seiner Wasseraufnahme imprägniert. Nach 16-stündiger Trocknung erfolgte eine zweite Imprägnierung, zu der 5 g Ir(CH₃COO)₃ in Wasser gelöst und auf 21,5 ml Gesamtlösung ergänzt wurden. Damit wurde das Material ein zweites mal entsprechend seiner Wasseraufnahme imprägniert und erneut 16 Stunden getrocknet und abschließen bei 250°C im Drehrohr kalziniert.

### Beispiel 1: Herstellung von optisch aktivem 1,2,4-Butantriol (BTO):

In einem diskontinuierlichen Autoklaven (300 ml Füllungsvermögen) wurden 5 g des Katalysators 1 mit 50 ml Wasser vorgelegt und bei 60 bar Wasserstoffdruck und 270°C für 2 Stunden gerührt. Anschließend wurden 24 g Apfelsäure (ÄS) und 120 g Wasser nachgefüllt und bei einem Druck von 230 bis 250 bar und einer Temperatur von 100°C über einen Zeitraum von 36 h hydriert. Der Reaktionsaustrag enthielt 67 mol-% 1,2,4-Butantriol (ee > 98,2), 22 mol-% Hydroxybutyrolacton (ee > 98,2), 5 mol-% Butandiol (BDO) und 5 mol-% nicht umgesetzte Äpfelsäure.

### Beispiele 2 - 8: Herstellung von optisch aktivem 1,2,4-Butantriol (BTO) unter Variation des Katalysators:

Beispiel 1 wurde unter Einsatz der in Tabelle 1 genannten Katalysatoren wiederholt und liefert die ebenfalls in Tabelle 1 aufgeführten Ergebnisse:

**Tabelle 1:**

| Beispiel | Katalysator | 1,2,4 BTO | ß-HGBL | BDO | ÄS |
|---|---|---|---|---|---|
| | | [mol-%] | [mol-%] | [mol-%] | [mol-%] |
| 2 | 3 | 51 | 0,3 | 35 | 1 |
| | | ee > 99,2 % | | | |
| 3 | 2 | 56 | - | 14,6 | 2,6 |
| | | ee > 98,6 % | | | |
| 4 | 4 | 70 | 0,5 | 1,46 | 0,5 |
| | | ee = 97,8 % | | | |
| 5^{*)} | 8 | 72 | 7 | 6 | 1 |
| | | ee > 98,5 % | | | |
| 6 | 9 | 48 | 5 | 32 | 2 |
| | | ee > 99,6 % | | | |
| 7 | 10 | 60 | 16 | 11 | 2 |
| | | ee > 98,2 % | | | |
| 8 | 11 | 39 | 21 | 9 | 7 |
| | | ee > 99,4 % | | | |

| | | | | | |
|---|---|---|---|---|---|
| ß-HGBL: β-Hydroxy-γ-butyrolacton, ÄS: Äpfelsäure, BDO: 1,4-Butandiol ^{*)} Die Hydrierung wurde bei einem Druck von 190 - 200 bar über einen Zeitraum von 70 h durchgeführt. | | | | | |

### Beispiel 9: Herstellung von β-Hydroxy-y-butyrolacton

In einem diskontinuierlichen Autoklaven (300 ml Füllungsvermögen) wurden 2 g Re₂O₇ und 0.8 g PtO₂ mit 50 ml Wasser vorgelegt und bei 60 bar Wasserstoffdruck und 270°C für 2 Stunden gerührt. Anschließend werden 24 g Äpfelsäure und 120 g Wasser eingefüllt und bei einem Druck von 230 bis 250 bar und einer Temperatur von 100°C über einen Zeitraum von 36 h hydriert. Der Reaktionsaustrag enthielt 5,9 mol-% 1,2,4-Butantriol, 69 mol-% hydroxybutyrolacton (ee > 99,0), 7.7 mol-% Butandiol und 27 mol-% nicht umgesetzte Äpfelsäure.

### Beispiel 10:

In einem diskontinuierlichen Autoklaven (300 ml Füllungsvermögen) wurden 4 g Re₂O₇ mit 50 ml Wasser vorgelegt und bei 60 bar Wasserstoffdruck und 270°C für 2 Stunden gerührt. Anschließend wurden 24 g Äpfelsäure und 120 g Wasser zugefüllt und bei einem Druck von 230 bis 250 bar und einer Temperatur von 100°C Ober einen Zeitraum von 36 h hydriert Der Reaktionsaustrag enthielt 6,4 mol-% 1,2,4-Butantriol, 33 mol-% Hydroxy-γ-butyrolacton (ee > 99,0), 1 mol-% Butandiol, 43 mol-% nicht umgesetzte Äpfelsäure.

### Beispiele 11 und 12: Kontinuierliche Hydrierung von Äpfelsaure:

Im Rahmen einer kontinuierlichen Festbetthydrierung wurden 190 ml des Katalysators 5 in einem Festbettreaktor vorgelegt. Bei einem Druck von 200 bar und einer Temperatur von 100 °C wurden in Rieselfahrweise und bei geradem Durchgang eine 6 Gew.-%ige wässrige Lösung von Äpfelsäure (ÄS) über den Katalysator geleitet. Unter Variation der Katalysatorbelastung erhielt man die in Tabelle 2 zusammengestellten Resultate:

**Tabelle 2:**

| Beispiel | Belastung | 1,2,4-BTO | β-HGBL | 1,4-Butandiol | ÄS [mol-%] |
|---|---|---|---|---|---|
| | [kg ÄS/L Kat *h] | [mol-%] | [mol-%] | [mol-%] | |
| 11 | 0.05 | 53 | 12 | 12 | 7 |
| 12 | 0.025 | 58 | 1 | 12 | 2 |

### Beispiele 13 und 14 sowie Vergleichsbeispiele 1 und 2:

In einem diskontinuierlichen Autoklaven (300 ml Füllungsvermögen) wurden 5 g des in Tabelle 3 angegebenen Katalysators mit 50 ml Wasser vorgelegt und mit 60 bar Wasserstoffdruck und 270°C für 2 Stunden gerührt Anschließend wurden 24 g Äpfelsäure und 120 g Wasser nachgefüllt und bei einem Druck von 230 bis 250 bar und einer Temperatur von 160°C über einen Zeitraum von 36 h hydriert. Nach 36 h erfolgt eine Analyse der im Autoklaven befindlichen Gase. Es werden im Gasraum die in Tabelle 3 aufgeführten Komponenten nachgewiesen.

**Tabelle 3:**

| | | Komponenten im Gasraum[%]** | | | | |
|---|---|---|---|---|---|---|
| Beispiel | Katalysator | H₂ | Methan | Ethan | Propan | Butan |
| 9 | 2 | 95,8 | 2,7 | 0,8 | 0,5 | 0,6 |
| 10 | 7 | 97,7 | 0,3 | - - | 0,04 | 0,05 |
| Vergleichsbeispiel 1 | 8% Ru, 11% Re auf HSAG* | 85 | 9,5 | 1,2 | 3,4 | 2,5 |
| Vergleichsbeispiel 2*** | Ru/Re-Mohr | 94,1 | 2,5 | 0,8 | 1,1 | 1,7 |

| | | | | | | |
|---|---|---|---|---|---|---|
| *HSAG = High Surface Activated Graphite; **%-Angaben in Vol.-% (Abweichung der Summe zu 100 % bedingt durch Messverfahren); ***Hydriertemperatur 100°C | | | | | | |

### Beispiele 15 und 16 sowie Vergleichsbeispiel 3:

In einem diskontinuierlichen Autoklaven (300 ml Füllungsvermögen) wurden 5 g des in Tabelle 3 angegebenen Katalysators mit 50 ml Wasser vorgelegt und mit 60 bar Wasserstoffdruck und 270°C für 2 Stunden gerührt. Anschließend wurden 24 g Äpfelsäure und 120 g Wasser nachgefüllt und bei einem Druck von 230 bis 250 bar und einer Temperatur von 100°C über einen Zeitraum von 36 h hydriert. Nach 36 h erfolgt eine Analyse der im Autoklaven befindlichen Gase. Es werden im Gasraum die in Tabelle 4 aufgeführten Komponenten nachgewiesen.

**Tabelle 4:**

| Beispiel | Katalysator | H₂ | Methan | Ethan | Propan | Butan |
|---|---|---|---|---|---|---|
| 15 | 8 | 99,6 | 0,1 | 7 ppm | 20 ppm | 107 ppm |
| 16 | 9 | 98,8 | 0,1 | 17ppm | 58ppm | 554 ppm |
| Vergleichsbeispiel 3 | Ru/Re-Mohr | 98 | 304 ppm | 7 ppm | 8 ppm | 23 ppm |

### Beispiel 17: Herstellung von Alaninol

In einem diskontinuierlichen Autoklaven (300 ml Füllungsvermögen) wurden 5 g des Katalysators 6 mit 50 ml Wasser vorgelegt und bei 60 bar Wasserstoffdruck und 270°C für 2 Stunden gerührt. Anschließend werden 24 g L-Alanin, 100 g Wasser und 13,2 g H₂SO₄ nachgefüllt und bei einem Druck von 180 bis 200 bar und einer Temperatur von 100°C Ober einen Zeitraum von 12 h hydriert. Der Reaktionsaustrag enthielt 57 mol-% L-Alaninol (ee > 99,4) und 12 mol-% nicht umgesetztes L-Alanin.

### Beispiel 18: Herstellung von Alaninol

In einem diskontinuierlichen Autoklaven (300 ml Füllurigsvermögen) wurden 5 g des Katalysators 4 mit 50 ml Wasser vorgelegt und bei 60 bar Wasserstoffdruck und 270°C für 2 Stunden gerührt. Anschließend werden 24 g L-Alanin, 100 g Wasser und 13,2 g H₂SO₄ nachgefüllt und bei einem Druck von 180 bis 200 bar und einer Temperatur von 100°C über einen Zeitraum von 12 h hydriert, Der Reaktionsaustrag 43 mol-% L-Alaninol (ee > 99,4) und 40 mol-% nicht umgesetztes L-Alanin.

## Patentansprüche

1. Verfahren zur Herstellung optisch aktiver Hydroxy-, Alkoxy-, Amino-, Alkyl-, Aryl- oder Chlor-substituierter Alkohole oder Hydroxycarbonsäuren mit 3 bis 25 Kohlenstoffatomen oder deren Säurederivate oder Cyclisierungsprodukte durch Hydrierung der entsprechend substituierten optisch aktiven Mono- oder Dicarbonsäuren oder deren Säurederivate in Gegenwart eines Katalysators, dessen Aktivkomponente aus Rhenium besteht oder Rhenium und mindestens ein weiteres Element ausgewählt aus der Gruppe der Elemente Pd, Pt, Rh, Ir, Cu, Ag, Au, Cr, Mo, W, Co und Ni enthält, mit der Maßgabe dass im Falle der Herstellung optisch aktiver 2-Amino-, 2-Chlor-, 2-Hydroxy- und 2-Alkoxy-1-Alkanole durch katalytische Hydrierung entsprechender optisch aktiver 2-Amino-, 2-Chlor-, 2-Hydroxy- und 2-Alkoxycarbonsäuren oder ihrer Säurederivate das mindestens eine weitere Element nicht Palladium oder Platin ist.

2. Verfahren nach Anspruch 1, **dadurch gekennzeichnet, dass** das mindestens eine weitere Element ausgewählt ist aus der Gruppe der Elemente Pd, Pt, Rh und Ir.

3. Verfahren nach Anspruch 1, **dadurch gekennzeichnet, dass** das mindestens eine weitere Element ausgewählt ist aus der Gruppe der Elemente: Rh, Ir, Cu, Ag, Au, Co und Ni.

4. Verfahren nach Anspruch 3, **dadurch gekennzeichnet, dass** das mindestens eine weitere Element Rh oder Ir ist.

5. Verfahren nach Anspruch 1 bis 4, **dadurch gekennzeichnet, dass** man optisch aktive Mono- oder Dicarbonsäuren oder deren Säurederivate umsetzt, die mindestens ein Stereozentrum in α- oder β-Position zu mindestens einer zu hydrierenden Carbonsäurefunktion oder davon abgeleiteten Säurederivatfunktion aufweisen.

6. Verfahren nach Anspruch 1 zur Herstellung
a. optisch aktiver 3-Hydroxy-, 3-Alkoxy-, 3-Amino-, 3-Alkyl-, 3-Aryl- oder 3-Chlor-1-Alkanolen ausgehend von den entsprechend substituierten optisch aktiven 3-Hydroxy-, 3-Alkoxy-, 3-Amino-, 3-Alkyl-, 3-Aryl- oder 3-Chlor-Monocarbonsäuren oder deren Säurederivaten oder
b. optisch aktiver Hydroxy-, Alkoxy-, Amino-, Alkyl-, Aryl- oder Chlor-substituerter Diole oder Triole oder deren Cyclisierungsprodukte ausgehend von den entsprechend substituierten optisch aktiven Dicarbonsäuren oder deren Säurederivate durch Hydrierung beider Carbonsäurefunktionen oder
c. optisch aktiver Alkyl- oder Aryl-substituierter Alkanole ausgehend von den entsprechend substituierten optisch aktiven Alkyl- oder Aryl-substituierten Monocarbonsäuren
in Gegenwart eines Katalysators, dessen Aktivkomponente Rhenium und Palladium oder Rhenium und Platin enthält.

7. Verfahren nach den Ansprüchen 1 bis 6, **dadurch gekennzeichnet, dass** man die Katalysatoren in geträgerter Form einsetzt.

8. Verfahren nach Anspruch 7, **dadurch gekennzeichnet, dass** man Katalysatoren einsetzt, die, jeweils bezogen auf das Gesamtgewicht des fertigen Katalysators und gerechnet als Metall, 0,01 bis 50 Gew.-% Rhenium und 0,01 bis 30 Gew.-% des mindestens einen weiteren Metalls ausgewählt aus der Gruppe der Elemente Pd, Pt, Rh, Ir, Cu, Ag, Au, Cr, Mo, W, Co und Ni einsetzt.

9. Verfahren nach Anspruch 7 und 8, **dadurch gekennzeichnet, dass** man als Trägermaterial ZrO₂, TiO₂, Al₂O₃, SiO₂, Aktivkohle, Ruße, Graphite oder High Surface Area Graphite einsetzt.

10. Verfahren nach Anspruch 9, **dadurch gekennzeichnet, dass** man Rhenium und das mindestens eine weitere Element ausgewählt aus der Gruppe der Elemente Pd, Pt, Rh, Ir, Cu, Ag, Au, Cr, Mo, W, Co und Ni in Gegenwart eines Reduktionsmittels auf den Träger aufbringt.

11. Verfahren nach Anspruch 1 bis 4 zur Herstellung von 1,2-Propandiol, 1,2-Butandiol, 1,2-Pentandiol, 1,3-Pentandiol, Leucinol, Isoserinol, Valinol, Isoleucinol, Serinol, Threoninol, Lysinol, Phenylalaninol, Tyrosinol, Prolinol, 2-Chlorpropanol, 2-Methyl-1-butanol, 1,2,4-Butantriol, 1,2,5-Pentantriol, 1,2,6-Hexantriol 2,3-Dimethylbutan-1,4-diol, 2-Methylbutan-1,4-diol, 2-Hydroxy-γbutyrolacton, 3-Hydroxy-γ-butyrolacton, 2-Chlor-γ-butyrolacton, 3-Chlor-γbutyrolacton, 2-Amino-γ-butyrolacton, 3-Amino-γ-butyrolacton, 2-Methyl-γbutyrolacton, 3-Methyl-γ-butyrolacton, 3-Hydroxy-δ-valerolacton, 4-Hydroxy-5-valerolacton, 2-Hydroxy-Tetrahydrofuran, 2-Methyl-Tetrahydrofuran oder 2-Amino-Tetrahydrofuran.

12. Verfahren nach Anspruch 1 bis 11, **dadurch gekennzeichnet, dass** man die Hydrierung bei einem Druck von 100 bis 300 bar durchführt.

13. Verfahren nach Anspruch 1 bis 12, **dadurch gekennzeichnet, dass** man die Hydrierung bei einer Temperatur von 30 bis 180°C durchführt.

14. Verfahren nach Anspruch 1 bis 13, **dadurch gekennzeichnet, dass** man die Hydrierung in Gegenwart einer Säure durchführt.

## Claims

1. A process for preparing optically active hydroxy-, alkoxy-, amino-, alkyl-, aryl- or chlorine-substituted alcohols or hydroxy carboxylic acids having from 3 to 25 carbon atoms or their acid derivatives or cyclization products by hydrogenating the correspondingly substituted optically active mono- or dicarboxylic acids or their acid derivatives in the presence of a catalyst whose active component consists of rhenium or of rhenium and comprises at least one further element selected from the group of the elements Pd, Pt, Rh, Ir, Cu, Ag, Au, Cr, Mo, W, Co and Ni, with the proviso that, in the case of the preparation of optically active 2-amino-, 2-chloro-, 2-hydroxy- and 2-alkoxy-1-alkanols by catalytically hydrogenating corresponding optically active 2-aminocarboxylic acids, 2-chlorocarboxylic acids, 2-hydroxycarboxylic acids and 2-alkoxycarboxylic acids or their acid derivatives, the at least one further element is not palladium or platinum.

2. The process according to claim 1, wherein the at least one further element is selected from the group of the elements Pd, Pt, Rh and Ir.

3. The process according to claim 1, wherein the at least one further element is selected from the group of the elements: Rh, Ir, Cu, Ag, Au, Co and Ni.

4. The process according to claim 3, wherein the at least one further element is Rh or Ir.

5. The process according to claim 1 to 4, wherein optically active mono- or dicarboxylic acids or their acid derivatives are converted which have at least one stereocenter in the α- or β-position to at least one carboxylic acid function or acid derivative function derived therefrom to be hydrogenated.

6. The process according to claim 1 for preparing
a. optically active 3-hydroxy-, 3-alkoxy-, 3-amino-, 3-alkyl-, 3-aryl- or 3-chloro-1-alkanols starting from the correspondingly substituted optically active 3-hydroxy-, 3-alkoxy-, 3-amino-, 3-alkyl-, 3-aryl- or 3-chloro-monocarboxylic acids or their acid derivatives or
b. optically active hydroxy-, alkoxy-, amino-, alkyl-, aryl- or chlorine-substituted diols or triols or their cyclization products selected from the correspondingly substituted optically active dicarboxylic acids or their acid derivatives by hydrogenating both carboxylic acid functions or
c. optically active alkyl- or aryl-substituted alkanols starting from the correspondingly substituted optically active alkyl- or aryl-substituted monocarboxylic acids
in the presence of a catalyst whose active component comprises rhenium and palladium or rhenium and platinum.

7. The process according to claims 1 to 6, wherein the catalysts are used in supported form.

8. The process according to claim 7, wherein catalysts are used which, based in each case on the total weight of the finished catalyst and calculated as the metal, contain from 0.01 to 50% by weight of rhenium and from 0.01 to 30% by weight of the at least one further metal selected from the group of the elements Pd, Pt, Rh, Ir, Cu, Ag, Au, Cr, Mo, W, Co and Ni.

9. The process according to claims 7 and 8, wherein the support material used is ZrO₂, TiO₂, Al₂O₃, SiO₂, activated carbon, carbon blacks, graphites or high surface area graphite.

10. The process according to claim 9, wherein the rhenium and the at least one further element selected from the group of the elements Pd, Pt, Rh, Ir, Cu, Ag, Au, Cr, Mo, W, Co and Ni is applied to the support in the presence of a reducing agent.

11. The process according to claim 1 to 4 for preparing 1,2-propanediol, 1,2-butanediol, 1,2-pentanediol, 1,3-pentanediol, leucinol, isoserinol, valinol, isoleucinol, serinol, threoninol, lysinol, phenylalaninol, tyrosinol, prolinol, 2-chloropropanol, 2-methyl-l-butanol, 1,2,4-butanetriol, 1,2,5-pentanetriol, 1,2,6-hexanetriol, 2,3-dimethylbutane-1,4-diol, 2-methylbutane-1,4-diol, 2-hydroxy-γ-butyrolactone, 3-hydroxy-γ-butyrolactone, 2-chloro-γ-butyrolactone, 3-chloro-γ-butyrolactone, 2-amino-γ-butyrolactone, 3-amino-γ-butyrolactone, 2-methyl-γ-butyrolactone, 3-methyl-γ-butyrolactone, 3-hydroxy-δ-valerolactone, 4-hydroxy-δ-valerolactone, 2-hydroxytetrahydrofuran, 2-methyltetrahydrofuran or 2-aminotetrahydrofuran.

12. The process according to claim 1 to 11, wherein the hydrogenation is carried out at a pressure of from 100 to 300 bar.

13. The process according to claim 1 to 12, wherein the hydrogenation is carried out at a temperature of from 30 to 180°C.

14. The process according to claim 1 to 13, wherein the hydrogenation is carried out in the presence of an acid.

## Revendications

1. Procédé de fabrication d'alcools ou d'acides hydroxycarboxyliques optiquement actifs à substitution hydroxy, alcoxy, amino, alkyle, aryle ou chloro avec 3 à 25 atomes de carbone ou de leurs dérivés acides ou de leurs produits de cyclisation par hydrogénation des acides mono- ou dicarboxyliques optiquement actifs substitués correspondants ou de leurs dérivés acides en présence d'un catalyseur dont le composant actif est constitué de rhénium ou contient du rhénium et au moins un autre élément choisi dans le groupe des éléments Pd, Pt, Rh, Ir, Cu, Ag, Au, Cr, Mo, W, Co et Ni, à condition qu'en cas de fabrication de 2-amino-, 2-chloro-, 2-hydroxy- et 2-alcoxy-1-alcanols optiquement actifs par hydrogénation catalytique des acides 2-amino-, 2-chloro-, 2-hydroxy- et 2-alcoxycarboxyliques optiquement actifs correspondants ou de leurs dérivés acides, ledit élément supplémentaire ne soit pas le palladium ou le platine.

2. Procédé selon la revendication 1, **caractérisé en ce qu'**au moins un élément supplémentaire est choisi dans le groupe des éléments Pd, Pt, Rh et Ir.

3. Procédé selon la revendication 1, **caractérisé en ce qu'**au moins un élément supplémentaire est choisi dans le groupe des éléments : Rh, Ir, Cu, Ag, Au, Co et Ni.

4. Procédé selon la revendication 3, **caractérisé en ce qu'**au moins un élément supplémentaire est Rh ou Ir.

5. Procédé selon les revendications 1 à 4, **caractérisé en ce que** des acides mono- ou dicarboxyliques optiquement actifs ou leurs dérivés acides qui présentent au moins un centre stéréochimique en position α ou β par rapport à au moins une fonction acide carboxylique à hydrogéner ou une fonction dérivé acide dérivée de celle-ci sont mis en réaction.

6. Procédé selon la revendication 1 pour la fabrication
a. de 3-hydroxy-, 3-alcoxy-, 3-amino-, 3-alkyl-, 3-aryl- ou 3-chloro-1-alcanols optiquement actifs issus des acides 3-hydroxy-, 3-alcoxy-, 3-amino-, 3-alkyl-, 3-aryl- ou 3-chloro-monocarboxyliques optiquement actifs correspondants ou de leurs dérivés acides ou
b. de diols ou triols optiquement actifs à substitution hydroxy, alcoxy, amino, alkyle, aryle ou chloro ou de leurs produits de cyclisation issus des acides dicarboxyliques optiquement actifs substitués correspondants ou de leurs dérivés acides par hydrogénation des deux fonctions acide carboxylique ou
c. d'alcanols optiquement actifs à substitution alkyle ou aryle issus des acides monocarboxyliques substitués optiquement actifs correspondants à substitution alkyle ou aryle
en présence d'un catalyseur dont le composant actif contient du rhénium et du palladium ou du rhénium et du platine.

7. Procédé selon les revendications 1 à 6, **caractérisé en ce que** les catalyseurs sont utilisés sous forme supportée.

8. Procédé selon la revendication 7, **caractérisé en ce que** des catalyseurs qui contiennent, à chaque fois par rapport au poids total du catalyseur fini et calculé en tant que métal, 0,01 à 50 % en poids de rhénium et 0,01 à 30 % en poids d'au moins un métal supplémentaire choisi dans le groupe des éléments Pd, Pt, Rh, Ir, Cu, Ag, Au, Cr, Mo, W, Co et Ni sont utilisés.

9. Procédé selon les revendications 7 et 8, **caractérisé en ce que** du ZrO₂, du TiO₂, du Al₂O₃, du SiO₂, du charbon actif, des noirs de carbone, des graphites ou des graphites de grande surface sont utilisés en tant que matériau support.

10. Procédé selon la revendication 9, **caractérisé en ce que** du rhénium et au moins un élément supplémentaire choisi dans le groupe des éléments Pd, Pt, Rh, Ir, Cu, Ag, Au, Cr, Mo, W, Co et Ni sont placés sur le support en présence d'un réducteur.

11. Procédé selon les revendications 1 à 4 pour la fabrication de 1,2-propanediol, 1,2-butanediol, 1,2-pentanediol, 1,3-pentanediol, leucinol, isosérinol, valinol, isoleucinol, sérinol, thréoninol, lysinol, phénylalaninol, tyrosinol, prolinol, 2-chloropropanol, 2-méthyl-1-butanol, 1,2,4-butanetriol, 1,2,5-pentanetriol, 1,2,6-hexanetriol, 2,3-diméthylbutan-1,4-diol, 2-méthylbutan-1,4-diol, 2-hydroxy-γ-butyrolactone, 3-hydroxy-γ-butyrolactone, 2-chloro-γ-butyrolactone, 3-chloro-γ-butyrolactone, 2-amino-γ-butyrolactone, 3-amino-γ-butyrolactone, 2-méthyl-γ-butyrolactone, 3-méthyl-γ-butyrolactone, 3-hydroxy-δ-valérolactone, 4-hydroxy-δ-valérolactone, 2-hydroxy-tétrahydrofurane, 2-méthyl-tétrahydrofurane ou 2-amino-tétrahydrofurane.

12. Procédé selon les revendications 1 à 11, **caractérisé en ce que** l'hydrogénation est réalisée à une pression de 100 à 300 bars.

13. Procédé selon les revendications 1 à 12, **caractérisé en ce que** l'hydrogénation est réalisée à une température de 30 à 180 °C.

14. Procédé selon les revendications 1 à 13, **caractérisé en ce que** l'hydrogénation est réalisée en présence d'un acide.
